(19) Europäisches Patentamt
European Patent Office
Office européen des brevets

(11) EP 0 552 223 B1

(12) EUROPEAN PATENT SPECIFICATION

(45) Date of publication and mention of the grant of the patent:
**17.07.1996 Bulletin 1996/29**

(21) Application number: **91917870.7**

(22) Date of filing: **10.10.1991**

(51) Int Cl.[6]: **G01N 33/48**, G01N 27/327

(86) International application number:
**PCT/DK91/00308**

(87) International publication number:
**WO 92/07263 (30.04.1992 Gazette 1992/10)**

# (54) USE OF BENZENE DERIVATIVES AS CHARGE TRANSFER MEDIATORS

VERWENDUNG VON BENZENDERIVATEN ALS CHARGE-TRANSFER-MEDIATOREN

UTILISATION DE DERIVES BENZENIQUES COMME MEDIATEURS DE TRANSFERT DE CHARGE

(84) Designated Contracting States:
**AT BE CH DE DK ES FR GB GR IT LI LU NL SE**

(30) Priority: **10.10.1990 DK 2451/90**
**25.04.1991 DK 773/91**

(43) Date of publication of application:
**28.07.1993 Bulletin 1993/30**

(73) Proprietor: **NOVO NORDISK A/S**
**DK-2880 Bagsvaerd (DK)**

(72) Inventors:
- **BUCH-RASMUSSEN, Thomas**
**DK-2820 Gentofte (DK)**
- **OLSEN, Bjarke, Rosenblad**
**DK-2660 Brondby Strand (DK)**
- **KULYS, Juozas, Institute of Biochemistry**
**Vilnius-MTP (SU)**
- **BECHGAARD, Klaus**
**DK-2610 Rodovre (DK)**
- **CHRISTENSEN, Jorn, B.**
**DK-2200 Copenhagen N (DK)**
- **WANG, Joseph**
**Las Cruces, NM 88001 (US)**
- **OZSOZ, Mehmet (Sengun)**
**Karsiyaka, Izmir (TR)**
- **COLIN, Fernan,**
**Univ. de Médecine et de Pharmacie**
**B-Bruxelles (BE)**
- **GARCIA, Orfélia,**
**Univ. de Médecine et de Pharmacie**
**B-Bruxelles (BE)**

(56) References cited:
**EP-A- 0 125 137** **WO-A-89/10395**
**WO-A-90/10861**

- **CHEMICAL ABSTRACTS, vol. 92, no. 15, 14 April 1980, Columbus, OH (US); A. BACCARINI-MELANDRI et al., p. 378, no. 125207d/**
- **JOURNAL OF ANALYTICAL CHEMISTRY OF THE USSR, part 1, vol. 45, no. 9, 1990; J.**
- **LABUDA, pp. 445-455/**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 552 223 B1

Printed by Jouve, 75001 PARIS (FR)

## Description

### FIELD OF THE INVENTION

The present invention relates to a biochemical method for measuring the concentration of at least one constituent of a fluid sample, especially glucose in a glucose containing aqueous medium, for example a body fluid, in particular a whole blood sample and to charge transfer mediators for use in such a method.

### DESCRIPTION OF PRIOR ART

Electrode devices and methods for detecting the amount of selected compounds in a liquid mixture are known in the art. Thus, in European patent application having publication No. 78,636B and 125,137A a sensor electrode for in vivo measurement of the amount of a component in a liquid mixture is described. Therein, a charge transfer mediator, such as ferrocene, is used in an enzyme catalyzed reaction to transfer electrons arising from the reaction between the enzyme and a component in the liquid. The charge transfer mediator (hereinafter for the sake of brevity designated mediator) is adhered to the surface of the electrode in the form of a thin film layer or strip, for example by deposition from a solution. This known device suffers from the severe drawback that the mediator during the measurement rather rapidly will be depleted from the strip, causing a deterioration in measurement reproducibility. A similar device is described in European patent application having publication No. 127,958A. The electrode devices are preferably intended for in vivo measurement of glucose in blood.

International patent application having publication No. WO 89/10395 describes an enzyme electrode comprising a matrix made from a conductive powder, an enzyme and a mediator. The device suffers from the drawback that the concentration of the mediator is rather low. It is therefore necessary to operate the electrode at a rather high applied potential, leading to interference from other oxidizable substances in the sample.

Chemically modified electrodes are described in J.Anal.Chem. USSR 45 (1990), 445 et seq. According to Table 2 therein, the modifier could, for example, be tetramethylphenylenediamine (hereinafter designated TMPD) when the substance to be measured is ascorbic acid or NAD. Unfortunately, TMPD does not have a sufficient stability.

International Patent Application No. PCT/DK90/00067 describes a method of measuring at least one constituent of a fluid sample by means of an electrode device including at least one measuring electrode having an exposed surface part at a free end portion of the electrode device and extending longitudinally through said end portion, comprising removing an outer end section of the free end portion so as to provide a new exposed surface part of the measuring electrode, and subsequently exposing said exposed surface part of the measuring electrode to a sample of said fluid, so as to generate a measuring signal representative of said constituent of the sample.

The method according to International Patent Application No. PCT/DK90/00067 can be used for measuring the concentration of a constituent of gaseous and liquid fluid samples of any type. The method may in particular be used for measurements on fluid samples within the medical and veterinary field. Thus, the fluid sample may, for example, be a sample of animal or human body fluids, such as whole blood. The known method is especially useful in connection with the measurement of the content of glucose in blood and is based on an electrochemical principle using a mediator present in a non-polar medium in a sensor electrode which operates at a constant potential which is sufficiently low to avoid interference resulting from oxidation of other oxidizable substances, such as acetylsalicylic acid (aspirin), paracetamol (acetaminophen), ascorbic acid, and sulfonylureas (tolbutamide, glibenclamide), present in the sample. The method is especially advantageous in that a specific measure of the concentration of glucose is obtained without any significant uncertainty arising from a co-determination of interfering substances which, in general, can be present in the blood. The method is based on monitoring the flow of current at an electrically conductive sensor electrode when glucose oxidase catalyzes a redox reaction of glucose in the presence of a mediator.

International Patent Application No. PCT/DK90/00067 also describes an electrode device comprising an electrode body member (electrode device body) having a free end portion, and at least one measuring or sensor electrode extending axially through said end portion and having a substantially uniform cross-sectional area within said end portion. Because the cross-sectional area of each of the sensor or measuring electrodes positioned in the free end portion of the electrode body member is substantially uniform, the exposed active surface area of each electrode remains substantially unchanged when an outer end section or slice is removed from the free end portion. The electrode body member in which the measuring electrode is embedded may in itself define an outer electrode provided that this outer electrode is sufficiently electrically insulated from the other inner electrode or electrodes.

The electrode device comprises an electrode device body, a sensor electrode, a reference electrode and a suitable counter electrode. The electrodes are positioned in separate channels in the electrode device body having the shape of a cylindrical rod, and the electrode channels are longitudinally disposed in the electrode device body.

The body of the electrode device is made of a sliceable polymeric material and the sensor electrode ingredients are uniformly dispersed in a substantially water-insoluble carrier medium and uniformly distributed throughout the channels in the form of a sliceable, highviscosity paste such as paraffine. This construction permits the reuse of the electrode device for a number of determinations, a fresh sensing surface being provided before each determination by cutting away a cross-sectional slice. The measurement is performed by bringing a surface of the electrode device, comprising cross sections of all the electrodes present, into contact with a sample of the drop of whole blood, which is taken from a patient, preferably by the patient himself, before the measurement is to be made. Before every measurement, the sensing surface of the electrode device is renewed by cutting off a thin slice of the electrode device in order to ensure the removal of any contaminants present on the sensing surface and to provide a fresh sensing surface of the electrode device. In addition, the removal of the sensor electrode sensing surface which has been exposed to the aqueous medium in question during the preceding measurement serves to ensure that the required amount of glucose oxidase and mediator is present at the surface of the electrode(s).

The basis of the method for measuring the concentration of glucose in a sample of a glucose-containing aqueous medium, which can be performed by means of an apparatus comprising the above electrode device is the reaction between glucose and the enzyme glucose oxidase (GOD). Electrons produced by this oxidation reaction are transferred to a mediator and finally captured by a conductive material in the sensor electrode, for example graphite particles. The latter enzyme-catalyzed oxidation of glucose leads to gluconic acid and the "reduced form" of glucose oxidase, i.e. glucose oxidase wherein at least one functional group has been reduced. The reduced enzyme reacts with the oxidized form of the mediator, which is formed by donation of an electron from the "reduced" form of the mediator to the electrically conductive sensor electrode, and the glucose oxidase is thereby regenerated together with the reduced form of the mediator. The electrical current produced in the process is proportional to the concentration of glucose present in the sample.

The reactions can schematically be described by the following equations:

$$\text{glucose} + \text{glucose oxidase-FAD} + H_2O \rightarrow$$

$$\text{gluconic acid} + \text{glucose oxidase-FADH}_2 \qquad \text{(I)}$$

$$\text{glucose oxidase-FADH}_2 + 2\ CTM_{ox}^{+} \rightarrow$$

$$\text{glucose oxidase-FAD} + 2\ CTM_{red} + 2H^{+} \qquad \text{(II)}$$

$$2\ CTM_{red} \rightarrow 2\ CTM_{OX}^{+} + 2e^{-} \qquad \text{(III)}$$

where FAD designates the oxidized form of the flavin-adenine dinucleotide part of glucose oxidase, $FADH_2$ designates the reduced form of the flavin-adenine dinucleotide part of glucose oxidase, $CTM_{ox}^{+}$ designates the mediator in oxidized form and $CTM_{red}$ is the mediator in reduced form.

A simplified way of expressing the reactions is:

$$\text{glucose} + GOD \rightarrow \text{gluconic acid} + GOD^{-} \qquad \text{(IV)}$$

$$GOD^{-} + CTM_{ox}^{+} \rightarrow GOD + CTM_{red} \qquad \text{(V)}$$

$$CTM_{red} \rightarrow CTM_{ox}^{+} + e^{-} \qquad \text{(VI)}$$

Electrodes based on the above general principle are known, but they suffer from problems caused by the depletion of the mediator, due to relatively high water-solubility of the oxidized form thereof.

A significant extent of depletion of the mediator can result in several drawbacks, for example

(i) only one measurement can be performed in a given sample owing to rapid loss of the mediator to the medium of the sample;
(ii) the reproducibility of the measurement is poor as the extent of the depletion of the mediator is rather unpredictable and depends on the time elapsing between exposure of the sample to the electrodes in question and the performance of the actual measurement;
(iii) the sensitivity at low glucose concentrations is very poor; and
(iv) the applied potential between the sensor electrode and the reference electrode has to be rather high, leading to interference from other oxidizable substances present in the aqueous sample.

A prerequisite for a successful and reproducible measurement of the specific glucose concentration in a body fluid, in particular in whole blood, based on the above-mentioned principle using a suitable apparatus comprising the sensor electrode, a suitable reference electrode and a suitable counter electrode is thus that a sufficiently well-defined amount

of the mediator is present throughout the measurements.

The amount of the mediator present in the sensor electrode is a very important feature of the present invention. The amount should throughout the lifetime of the electrode device be sufficiently high to ensure that the concentration of the oxidized form of the mediator in the aqueous sample is not limiting the enzyme reaction during the measurement.

## DESCRIPTION OF THIS INVENTION

The present invention is related to a method based on the above mentioned electrochemical principle for measuring the concentration of certain compounds such as glucose, theophyllin, alcohol (ethanol), lactate or cholesterol, in a sample of an aqueous medium, for example a sample of body fluid, such as a sample of whole blood, blood plasma, serum, urine or saliva, in which certain groups of mediators are used. The mediators are characterized by having a low solubility in their reduced form in water, for example lower than 10 mM.

This ensures that the mediator is present and available in an amount sufficient to ensure that the concentration of its oxidized form is maintained substantially constant during the measurement.

Hence, this invention relates to a biochemical process involving electron transfer between a redox system and an electrode comprising a paste of electrically conductive particles and a pasting material and a charge transfer mediator, wherein the mediator has a low water solubility. In one aspect of this invention, the mediator has a high solubility in the pasting material. In the pasting material, the mediator may wholely or partially be present in crystalline form. The pasting material may be a non-polar oil such as paraffin oil.

The mediator should have the following characteristics:

1) A low solubility in water in the reduced form.
2) An oxidation potential between -300 and +400 mV versus an Ag/AgCl reference electrode.
3) The reaction rate of the mediator in the enzyme reaction and the electrode reaction must not be the limiting step in the total reaction rate.
4) It must be stable.
5) It must not participate in other reactions.

The present invention is also related to mediators for use in the method described above. Additionally, this invention relates to the use of compounds as mediators.

It has now, surprisingly, been found that compounds of the general formula X

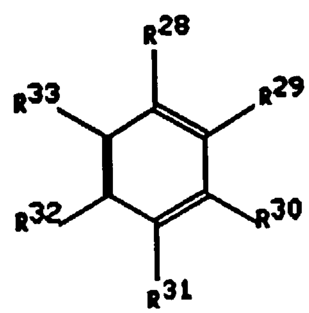

(X)

wherein $R^{28}$ represents morpholinyl or piperidyl, $R^{31}$ represents amino, alkylamino, dialkylamino, morpholinyl or piperidyl, $R^{29}$, $R^{30}$ and $R^{33}$ are the same or different and each represents hydrogen or alkyl, $R^{32}$ represents hydrogen, alkyl or nitro, or $R^{28}$ together with $R^{33}$ either represents a moiety of the formula Xa

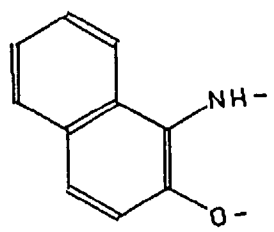

wherein the amino group is in the $R^{28}$ end, or $R^{28}$ together with $R^{33}$ represents a moiety of the formula Xb

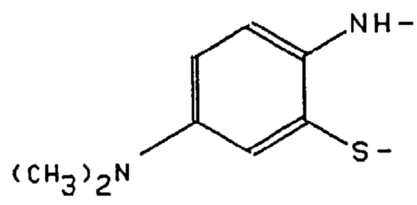

wherein the amino group is in the $R^{28}$ end, 1,4-diamino-2,3,5,6-tetramethylbenzene, 2,5-dimethyl-1,4-bis(dimethylamino)benzene, N-(4-morpholinophenyl)hexahydroazepine or Meldola's Blue (= 7-dimethylamino-1,2-benzophenoxazinium salts), fulfil the above, strict requirements for being a good mediator.

Especially preferred compounds of formula X are compounds wherein $R^{28}$ and $R^{31}$ are the same or different and each represents morpholinyl or piperidyl, and $R^{29}$, $R^{30}$, $R^{32}$ and $R^{33}$ are the same or different and each represents hydrogen or alkyl, preferably hydrogen.

Herein, the term alkyl is preferably lower alkyl which may be straight or branched and which preferably contains not more than 10 carbon atoms, more preferred not more than 6 carbon atoms. Analagous comments relates to groups containing an alkyl group such as alkylamino and dialkylamino.

Compounds of formula X are either known compounds or compounds which can be prepared in analogy with the preparation of known compounds.

The electrode device comprises a sensor electrode, a reference electrode and a counter electrode. In a preferred embodiment two sensor electrodes are present. The electrode comprises an electrically conductive material, together with glucose oxidase and the mediator. The electrically conductive material is a carbon-based material such as surface-oxidized graphite particles of a size of up to 50 µm, preferably 1 - 20 µm.

According to one aspect of this invention, the mediator has a low water solubility. If the mediator is in the reduced form in the paste, the mediator preferably has a solubility in water in the range from about 1 µM to about 10 mM. If the mediator is in the oxidized form in the paste, the mediator preferably has a solubility in water in the range from about 1 µM to about 100 mM.

The solubility of a certain mediator in the pasting material may be high, low or medium. Herein the statement that the mediator has a high solubility in the pasting material designates that the mediator is dissolved in the pasting material. Within the context of this invention, a high solubility of the mediator is a solubility of more than about 80 mM in the pasting material.

In a preferred embodiment of this invention, the mediator is wholely or partially present in crystalline form in the pasting material. This is the case when the mediator has a low or medium solubility in the pasting material. Within the context of this invention, a low solubility of the mediator is a solubility of less than about 1 mM in the pasting material. Surprisingly, the use of mediators in crystalline form give superior results. Preferably, homogeneous crystals are used.

In a preferred embodiment, the electrically conductive material is a carbon-based paste containing a pasting material. Useful pasting materials are non-polar substances which are substantially immiscible with water, such as paraffin, paraffin oil, silicone oil etc., paraffin oil being preferred.

The pasting material constitutes a support and a dispersion medium for the mediator and regulates the release of the mediator in a controlled way during the measurement. Furthermore, the pasting material serves as a cohesive medium by filling the interstices between the graphite particles.

The enzyme preferred in the method according to the invention is glucose oxidase, which is an oxidoreductase enzyme of the classification EC 1.1.3.4. However, other enzymes which are oxidoreductases may be used, for example theophyllin oxidase, alcohol oxidase, lactate oxidase, cholesterol oxidase, glucose dehydrogenase, L-amino acid oxidase or a glycolate oxidase.

The glucose oxidase will be attached to the surface-oxidized graphite particles and/or immobilized on the latter particles by means of a treatment involving the use of a carbodiimide reagent such as, for example, 1-cyclohexyl-3-(2-morpholinoethyl)carbodiimide metho-p-toluenesulfonate (= N-cyclohexyl-N'-β-[(N-methylmorpholino)ethyl]carbodiimide p-toluenesulfonate salt). The glucose oxidase is present in the sensor electrode in an amount of at least 500 IU per gram of graphite, preferably in an amount of at least about 1000 IU, such as at least about 2000 IU, more preferably at least about 4000 IU, most preferably at least about 6000 IU per gram of graphite, and in particular about 8000 IU per gram graphite.

An essential feature of the sensor electrode is that the glucose oxidase and the mediator are uniformly or homogeneously distributed in the carbon-containing paste. In this way it is possible to cut off a thin, cross-sectional slice of the electrode and at the same time ensure that the correct and sufficient amount of glucose oxidase and mediator is present at the working surface of the electrode, the working surface of the sensor electrode being the surface which is exposed to the glucose-containing aqueous medium, such as a blood sample.

In a preferred embodiment of the sensor electrode for determination of glucose, two sensor electrodes are present

in the electrode device. The two sensor electrodes can be similar or different. When the sensor electrodes are different, one electrode comprises the glucose oxidase in an active form and the other comprises the glucose oxidase in an inactive form which has been inactivated by heat denaturation at 50°C for 24 hours.

The advantage of using two similar sensor electrodes for the measurements is that it is possible in this way to obtain two responses, one from each sensor electrode. The responses should be substantially equal if the measurement is performed correctly. Different responses from two similar electrodes is an indication of an unreliable measurement due, for example, to inadequate contact between the sample and the electrodes.

The advantage of using two different sensor electrodes, one containing the active enzyme and the other one containing the inactivated enzyme, is that it is possible in this way to obtain a blank response from the electrode containing the inactivated enzyme, whereby response from interfering substances can be subtracted from the measurement performed at the sensor electrode containing the active glucose oxidase.

In the method according to the invention, the measurement of the concentration of glucose in a sample of an aqueous medium is performed at a potential between the sensor electrode and a reference electrode in communication with the sample of the aqueous medium which is in a range which is about 0 - 250 mV when the reference electrode is an Ag/AgCl reference electrode working at a concentration of chloride ions of about 145 meq/l, or, when the reference electrode is different therefrom, which is in a range corresponding to a range of about 0 - 250 mV measured versus an Ag/AgCl reference electrode working at a concentration of chloride ions of about 145 meq/l.

A sensor electrode for the determination of glucose may be prepared in the following manner:

The carbon particles, preferably graphite particles, are surface oxidized by heating, for example, at 100°C for a predetermined period in a well ventilated, continuously rotated vessel in the presence of dry atmospheric air. The surface-oxidized graphite particles are activated with 1-cyclohexyl-3-(2-morpholinoethyl) carbodiimide metho-p-toluenesulfonate. The activation is performed by mixing the graphite particles with the 1-cyclohexyl-3-(2-morpholinoethyl) carbodiimide metho-p-toluenesulfonate dissolved in an aqueous buffer solution, for example, an acetate buffer of pH value 4.76, followed by continuous stirring for about 2 - 4 hours at room temperature. After stirring, the mixture is washed several times (for example 4 - 7 times) with distilled water until an approximately neutral pH value (for example 5 - 7), and the carbodiimide-activated graphite particles are dried by evaporation of the water, for example by forced evaporation using a fan, or under reduced pressure, at room temperature or by lyophilization. Before immobilization of the glucose oxidase on the carbodiimide-activated graphite, the glucose oxidase is dissolved in an aqueous buffer having a suitable pH value at which the enzyme is sufficiently stable, and containing a coupling reagent for coupling the enzyme to the carbodiimide-activated graphite, for example a phosphate buffer having a pH value of 7.3 and containing 4 % (weight/weight) glutaraldehyde (i.e. glutaric dialdehyde). The immobilization is performed by suspending the carbodiimide-activated graphite particles in the buffer containing the enzyme and the coupling reagent. The suspension is stirred continuously for about 16 hours at 4°C followed by removal of the water by evaporation, for example at reduced pressure, at room temperature or by lyophilization. Using glutaraldehyde as coupling reagent the enzyme, preferably glucose oxidase, becomes covalently bonded to the graphite particles. The enzyme may, however, also be physically adsorbed to the surface of the particles.

In the following the term "GOD-containing graphite" is used to mean carbodiimide-activated graphite particles comprising the glucose oxidase, the particles being prepared in the manner described above.

The GOD-containing graphite is sieved through a 48 mesh (mesh size corresponding to a sieve having an opening of 297 μm) and the sieved material is then mixed with a sufficient amount of the non-polar pasting material, i.e. paraffin oil, in which the mediator has been dissolved. The amount of the mediator in the paraffin oil is larger than the required and final amount in the sensor electrode. The reason for this is that it should be possible to obtain a dispersion of GOD-containing graphite in the mediator solution having a consistency which allows the appropriate channel(s) in the electrode device body to be filled. The excess amount of the mediator solution is easily removed after filling of the channel (s) of the sensor electrode in the electrode device body by centrifugation.

To fill the sensor electrode channels(s), the electrode device body is placed in a special holder having a small funnel-shaped reservoir at the top. The dispersion containing the GOD-containing graphite and the mediator in paraffin oil solution is then poured into the reservoir. The electrode device body equipped with the filled reservoir is then centrifuged at about 15,000 - 50,000 x g for about 5 - 30 minutes, the electrode body device being positioned in an inverted position during centrifugation (i.e. that end of the device from which slices later are removed being uppermost, the funnel-shaped reservoir being inserted in that end). By the centrifugation the air is displaced from the channels and the GOD-containing graphite particles sediment, the interstices between the particles thus being filled with the mediator in paraffin oil, and the excess amount of the mediator paraffin oil remaining as a supernatant in the upper part. After centrifugation the excess of the mediator is easily removed and the surface of the sensor electrode is levelled with the surface of the electrode device body by cutting off a suitable cross-sectional portion of the electrode device.

The advantages achieved by using the above-mentioned method of preparing and filling the sensor electrode channel(s) in the electrode device body is that the components of the sensor electrode(s) become packed uniformly or homogeneously in the graphite-containing paste, the interstitial spaces between the GOD-containing particles being

filled with the mediator in paraffin oil such that substantially no air bubbles remain within the sensor electrode(s).

Stability test

The stability of a paste with a certain mediator can be determined using the following method:

The mediator is dissolved or mixed with paraffin and, thereafter, the enzyme coupled to graphite is added. The mixture is homogenized giving a paste. This paste is filled into electrode holders of plast giving so-called sticks. These sticks are tested using 0 and 10 mM glucose standards in order to characterize the activity of these sticks in relation to glucose. Thereafter, the paste containing sticks are stored at a constant temperature, for example, 4, 20, 35 and 54°C. After a certain period of time, for example 1 month, the sticks are tested using the 0 and 10 mM glucose standards and the activities measured are compared with the activity measured at originally. Those mediators present in the sticks for which the change of measurements for glucose are the lowest, are considered to be the mediators most stable in the system (pasta) in question.

Standard methods for evaluation of mediators

1) A paste containing GOD-immobilized graphite, paraffin and mediator is prepared. If the mediator has a big solubility in paraffin then 10 mg of mediator is used per ml of paraffin. If the mediator has a little solubility in paraffin then 50 mg of crystals are added per ml of paraffin in order to obtain a homogeneous mixture before graphite is added. The paste obtained is filled into sticks.

2) The activity of the paste is determined in a cyclic voltametri equipment where the current is measured as a function of the potential (CV sweep). Five concentrations of glucose are measured, i.e., 0, 5, 10, 15 and 20 mM. Based upon the results obtained, the following is evaluated:

a) whether the mediator can be oxidized and reduced by the graphite electrode,
b) whether the mediator can be reduced of GOD,
c) the optimal potential for the electorde to be used,
d) whether a linear response as a function of the concentration of glucose can be obtained using the concentration of mediator in question,
e) whether the concentration of the mediator can be optimized.

3) The response as a function of time at the optimal potential is to be measured. The time interval within which the response represents a stable response for the concentration of glucose is to be evaluated.

4) The stability of the mediator in the paste question is determined.

The concentration of the mediator in the sample phase kan be determined using the method described in Bioelectrochemistry and Bioenergetics 8 (1981), 103 - 113.

Reference electrode

The reference electrode can be any physically suitable reference electrode of a conventional type, preferably a silver/silver chloride reference electrode or a variant thereof comprising an aqueous solution of potassium chloride (for example a 1 M solution) in a gel (for example an agarose gel) and at the bottom having a silver/silver chloride electrode. A silver/silver chloride electrode is preferred, the electrode comprising a silver wire on which a layer of silver chloride has been established by immersion in ferric chloride, or by an electrolytic treatment in 0.1 M hydrochloric acid. The silver wire is rather thin and has preferably a diameter of about 100 - 300 µm, in particular about 200 µm. Alternatively, the reference electrode can comprise an appropriate number of silver chloride coated silver wires, each wire having a diameter of about 20 - 100 µm, preferably about 30 µm.

Following removal of a cross-sectional slice of the electrode device prior to performing a measurement it is clear that the freshly exposed cross-sectional surface of the silver wire is no longer covered by silver chloride. However, upon exposure to a sample of an aqueous medium which contains at least a moderate concentration of chloride ions (such as whole blood or urine) the latter cross-sectional surface rapidly becomes coated with an adequate layer of silver chloride by reaction between the silver from the silver wire and the chloride present in the aqueous sample. The chloride present in the aqueous sample may arise from dissolution of silver chloride from the periphery of the exposed silver surface of the reference electrode itself and/or from the content of chloride ion in the sample. In the case where the aqueous sample itself contains only a small concentration of chloride ions it is thus advantageous to use a reference

electrode comprising a number of silver wires, whereby a greater surface area of the silver chloride from the periphery of the reference electrode surface is available to release chloride ions to the aqueous sample, resulting in the formation of the necessary layer of silver chloride on the cross-sectional surface of the silver wire.

Alternatively, the reference electrode may be made of any sliceable plastic material in which is embedded a powder comprising small silver and silver chloride particles.

The reference electrode is suitably prepared by inserting the silver wire(s) on which the silver chloride layer has been established into the reference electrode channel of the electrode device body. The silver/silver chloride electrode is then fixed into position in the channel by injecting, for example, two-component epoxy adhesive into the free space in the channel.

Counter electrode

The counter electrode is preferably a silver wire of diameter about 100 - 300 μm, preferably about 200 μm. It may also be made of any suitably electrically conductive sliceable polymer-based material, for example a plastic material, or an electrically conductive carbon-based paste, for example a graphite-based paste.

During the measurement, the counter electrode delivers an electrical current which counterbalances the current generated at the sensor electrode as a result of the electrochemical reaction at the surface exposed to the sample, and as a result a fixed, constant potential between the sensor electrode and the reference electrode can be maintained.

Operation of the sensor electrode of an electrode device according to the invention at relatively low and constant potential is very important if interference from oxidizable substances other than glucose present in the blood is to be avoided.

In addition, the measurement must be reproducible and accuracy should be high, particularly at low glucose concentrations (as would be the case for a patient in hypoglycemic coma). The patient himself should be able to check the correct functioning of the electrode device by using a single test solution and finally it should be possible to manufacture the sensor on a large scale to strict specifications.

This invention is further illustrated by the following examples which, however, are not to be construed as limiting.

**Example 1**

Glucose oxidase (GOD) (E.C.1.1.3.4.) was immobilized on graphite particles by the following procedure: 10 g of graphite was oxidized in air at 100°C for 24 hours. The surface oxidized graphite particles were activated with 500 mg of 1-cyclohexyl-3-(2-morpholinoethyl)carbodiimide metho-p-toluenesulfonate in 100 ml of 0.1 M acetate buffer (pH 4.76) for 2 hours, then washed with distilled water until an approximately neutral pH value and dried. The activated graphite was mixed with 30 ml of 0.01 phosphate buffer (pH 7.3) containing 80,000 IU GOD and 4% glutaraldehyde for 16 hours at 4°C, dried and sieved through a 48 mesh to obtain uniform particles.

1 g of paraffin oil containing 5% Methylene green (4-nitro-3,7-bis(dimethylamino)phenothiazine) was mixed carefully with 2 g of GOD modified graphite to a uniform pasta.

The pasta was filled into a plastic rod in which it was possible to cut a thin cross-sectional slice to introduce a new well-defined electrode area. Before each measurement, the electrode surface was renewed. The working electrode area used was 3 $mm^2$.

In the study of the working electrode, an Ag auxiliary electrode and an Ag/AgCl reference electrode was used. In a cyclic voltammogram, a catalytic current from the reoxidation of the mediator was observed beginning at -50 mV and being stable between 50 and 400 mV. The current was proportional to the concentration of glucose used.

Chronoamperometry was preformed at 100 mV using the integrated current from 30 to 60 seconds after applying the potential. A linearity up to 40 mM glucose was obtained. The current was 500 nA/(sec*mM). The signal was not affected by the oxygen concentration in the solution. Reference pasta without Methylene green was not affected by different glucose concentrations.

**Example 2**

Comparison between selected mediators.

Data in the table are obtained using the same procedure as in Example 1.

The following abbreviations are used: Methylene green = MG, Mendola blue = MB, 1-dimethylamino-4-morpholinobenzene = MDMP, 1,4-dimorpholinobenzene = BMP, 1,4-dipiperidinobenzene = BPP, 1-morpholino-4-piperidinobenzene = MPP.

| | MG | MB | MDMP | BMP | BPP | MPP |
|---|---|---|---|---|---|---|
| Concentration in pasta (%) | 5 | 5 | 2 | 2 | 0.5 | 2 |
| Redoxpotential at GCElectrode[1] | -149 | -175 | 124 | 246 | 141 | 166 |
| Catalytic interval beginning at (mV)[1] | -50 | -50 | -20 | 50 | 0 | 20 |
| Working potential[1] | 100 | 100 | 100 | 200 | 150 | 100 |
| Signal to noice ration[2] | 22.5 | 4.8 | 8.3 | 5.6 | 4.7 | 10 |
| Linearity in mM | 40 | 40 | 20 | 20 | 20 | 20 |
| SD% of 100 measurement[2] | 7.5 | 6.5 | 4.5 | 6.5 | 3.5 | 4.0 |

[1] Ag/AgCl ref.,
[2] at 7mM glucose.

**Example 3**

Stability test of selected mediators.

Data are obtained using the same basis procedure as in Example 1. Rods with selected mediators were stored at different temperature to accelerate processes which could give new analytical performance.

The same abbreviations as in Example 2 are used.

| Mediator: | | MG | | MDMP | | BMP | | BPP | | MP | |
|---|---|---|---|---|---|---|---|---|---|---|---|
| Time: | | 0 | 75 | 0 | 72 | 0 | 72 | 0 | 78 | 0 | 70 |
| Temp. | | | | | | | | | | | |
| 4°C | a | 13.7 | 13.2 | 17.2 | 16.9 | 20.2 | 21.2 | 12.3 | 12.4 | 11.7 | 8.8 |
| | b | 10.0 | 14.3 | 16.2 | 18.1 | 21.3 | 19.3 | 11.1 | 13.7 | 9.2 | 10.5 |
| 20°C | a | 14.9 | 14.3 | 16.4 | 17.4 | 18.5 | 21.4 | 10.0 | 11.0 | 10.0 | 11.9 |
| | b | 12.5 | 15.4 | 16.0 | 15.4 | 20.5 | 23.3 | 10.5 | 11.1 | 7.2 | 9.3 |
| 35°C | a | 12.1 | 11.2 | 15.4 | 15.7 | 20.0 | 18.0 | 9.1 | 8.7 | 7.0 | 9.0 |
| | b | 13.3 | 12.2 | 13.4 | 14.5 | 16.7 | 20.3 | 10.6 | 10.4 | 9.0 | 9.5 |
| 54°C | a | 15.8 | 10.4 | 17.4 | 6.9 | 18.4 | 15.0 | 11.1 | 6.7 | 12.5 | 4.1 |
| | b | 12.8 | 12.6 | 14.4 | 8.5 | 20.5 | 14.6 | 10.3 | 5.6 | 11.3 | 4.7 |

Taking the reproducebility from slice to slice and from rod to rod into account, it can be concluded that all pasta are stable within at least 70 days at 35°C. From the data at 54°C, it can be extrapolated that the stability at 4°C will be at least 2 years.

**Example 4**

Mediators for a theophylline assay.

Theophylline oxidase was obtained from GDS Diagnostics, Division of GDS Technology, Inc., 25235 Leer Drive, P.O. Box 773 Elkhart, IN 46515, USA. The GDS Enzymatic Theophylline Reagent kit was used. The reagent B which contained the enzyme Theophylline oxidase was dialyzed against a phosphate buffer at pH 7.0 and the enzyme was lyophilized and coupled to graphite as in Example 1. The mediators used in Example 2 and dimethylferrocene (DMF) and tetrathiafulvalene (TTF) were tested in the system. DMF and TTF were used in 0.1 M and 0.06 M in paraffin. DMF was measured with a potential at 110 mV vs. Ag/AgCl reference electrode and for TTF 60 mV was used. For the rest of the mediators the potentials as in Example 2 were used. The current density obtained differs from the glucose reaction in Example 1. Typically, the current density was in the range 1 - 10 $\mu A/cm^2/mmol$. An oxygen dependence was observed at low concentrations.

**Example 5**

Mediators for lactate and alcohol assays.

Lactate oxidase and alcohol oxidase were obtained from Sigma and the enzymes were used as in Example 1. The same mediators as in Example 4 were tested in the electrodes. The best response for the lactate system was obtained with Methylene green, which gave a current density at 4 µA/$cm^2$/mM at 100 mV vs. Ag/AgCl reference electrode. For the alcohol system ethanol was used as enzyme substrate. The current density was much lower than for the lactate system and none of the tested mediators worked optimally. Other mediators sucessfully tested with glucose oxidase are expected to give higher current densitites due to differences in hydrophobicity and hydrophillicity.

## Claims

1. A biochemical process for measuring the concentration of certain compounds in an aqueous medium which process involves electron transfer between a redox system and an electrode comprising a paste of electrically conductive particles and a pasting material and a charge transfer mediator, characterized in that the mediator is a compound of the general formula X

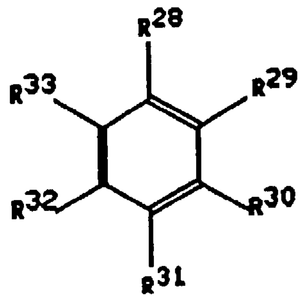

(X)

wherein $R^{28}$ represents morpholinyl or piperidyl, $R^{31}$ represents amino, alkylamino, dialkylamino, morpholinyl or piperidyl, $R^{29}$, $R^{30}$ and $R^{33}$ are the same or different and each represents hydrogen or alkyl, $R^{32}$ represents hydrogen, alkyl or nitro, or $R^{28}$ together with $R^{33}$ either represents a moiety of the formula Xa

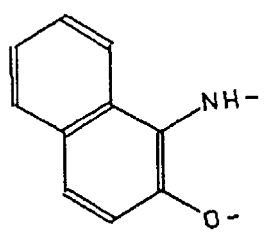

wherein the amino group is in the $R^{28}$ end, or $R^{28}$ together with $R^{33}$ represents a moiety of the formula Xb

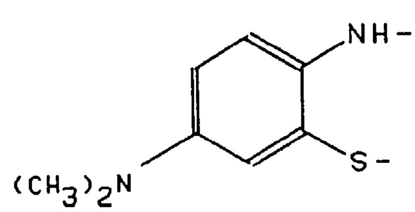

wherein the amino group is in the $R^{28}$ end, 1,4-diamino-2,3,5,6-tetramethylbenzene, 2,5-dimethyl-1,4-bis(dimethylamino)benzene, N-(4-morpholinophenyl)hexahydroazepine or Meldola's Blue (= 7-dimethylamino-1,2-benzophenoxazinium salts).

2. A process according to Claim 1, wherein one or more of the substituents designated $R^{29}$, $R^{30}$ and $R^{33}$ are hydrogen.

3. A process according to Claim 1 or 2, wherein $R^{32}$ is hydrogen or nitro.

4. Process, according to Claim 1, wherein the mediator is 1-dimethylamino-4-morpholinobenzene, 1,4-dimorpholinobenzene, 1-morpholino-4-piperidinobenzene, 1,4-dipiperidinobenzene or 4-nitro-3,7-bis(dimethylamino) phenothiazine (= methylene green).

5. Process, according to any one of the preceding claims, wherein the mediator wholely or partially is present in crystalline form in the pasting material.

6. Use of benzene derivatives with the general formula X stated in Claim 1 with the definitions of the symbols stated therein, 1,4-diamino-2,3,5,6-tetramethylbenzene, 2,5-dimethyl-1,4-bis(dimethylamino)benzene, N-(4-morpholinophenyl)hexahydroazepine or Meldola's Blue (= 7-dimethylamino-1,2-benzo- phenoxazinium salts) as a charge transfer mediator.

7. Use, according to Claim 6, of the mediators stated in Claim 2.

8. Use, according to Claim 6, of the mediators stated in Claim 3.

9. Use, according to Claim 6, of the mediators stated in Claim 4.

## Patentansprüche

1. Ein biochemisches Verfahren zur Messung der Konzentration bestimmter Verbindungen in einem wäßrigen Medium, wobei das Verfahren Elektronenübertragung zwischen einem Redoxsystem und einer Elektrode umfaßt, die eine Paste aus elektrisch leitfähigen Teilchen und einem pastenbildenden Material und einen Charge-transfer-Mediator umfaßt, dadurch gekennzeichnet, daß der Mediator eine Verbindung der allgemeinen Formel X

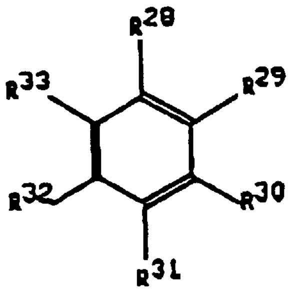

(X)

in der $R^{28}$ Morpholinyl oder Piperidyl darstellt, $R^{31}$Amino, Alkylamino, Dialkylamino, Morpholinyl oder Piperidyl darstellt, $R^{29}$, $R^{30}$ und $R^{33}$ identisch oder verschieden sind und jeweils Wasserstoff oder Alkyl'darstellen, $R^{32}$ Wasserstoff, Alkyl oder Nitro darstellt oder $R^{28}$ zusammen mit $R^{33}$ entweder eine Einheit der Formel Xa

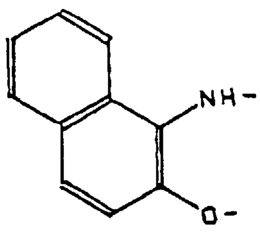

in der die Aminogruppe im $R^{28}$-Ende liegt, darstellt oder $R^{28}$ zusammen mit $R^{33}$ eine Einheit der Formel Xb

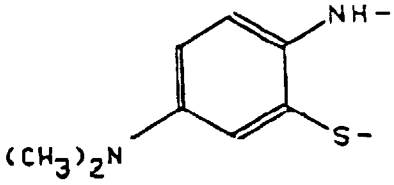

in der die Aminogruppe im $R^{28}$-Ende liegt, darstellt, 1,4-Diamino-2,3,5,6-tetramethylbenzen, 2,5-Dimethyl-1,4-bis (dimethylamino)benzen, N-(4-Morpholinophenyl)hexahydroazepin oder Meldolablau (= 7-Dimethylamino-1,2-benzophenoxaziniumsalze) ist.

2. Ein Verfahren nach Anspruch 1, wobei einer oder mehrere der Substituenten, die mit $R^{29}$, $R^{30}$ und $R^{33}$ bezeichnet sind, Wasserstoff sind.

3. Ein Verfahren nach Anspruch 1 oder 2, wobei $R^{32}$ Wasserstoff oder Nitro ist.

4. Verfahren nach Anspruch 1, wobei der Mediator 1-Dimethylamino-4-morpholinobenzen, 1,4-Dimorpholinobenzen, 1-Morpholino-4-piperidinobenzen, 1,4-Dipiperidinobenzen oder 4-Nitro-3,7-bis(dimethylamino)phenothiazin (= Methylengrün) ist.

5. Verfahren nach einem der vorangehenden Ansprüche, wobei der Mediator vollständig oder teilweise in kristalliner Form im pastenbildenden Material vorhanden ist.

6. Verwendung von Benzenderivaten mit der allgemeinen Formel X, die in Anspruch 1 angegeben ist, mit den darin angegebenen Definitionen der Symbole, 1,4-Diamino-2,3,5,6-tetramethylbenzen, 2,5-Dimethyl-1,4-bis(dimethylamino) benzen, N-(4-Morpholinophenyl)hexahydroazepin oder Meldolablau (= 7-Dimethylamino-1,2-benzophenoxaziniumsalze) als ein Charge-transfer-Mediator.

7. Verwendung nach Anspruch 6 der in Anspruch 2 angegebenen Mediatoren.

8. Verwendung nach Anspruch 6 der in Anspruch 3 angegebenen Mediatoren.

9. Verwendung nach Anspruch 6 der in Anspruch 4 angegebenen Mediatoren.

**Revendications**

1. Procédé biochimique pour la mesure de la concentration de certains composés dans un milieu aqueux, lequel procédé comprend le transfert d'électrons entre un système oxydoréducteur et une électrode comprenant une pâte de particules conductrices électriquement et un matériau de mise en pâte et un médiateur de transfert de charge, caractérisé en ce que le médiateur est un composé de la formule générale X

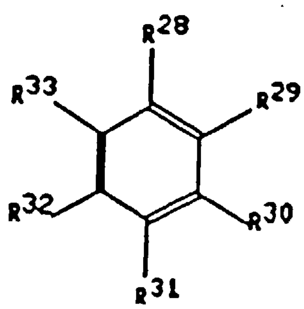

(X)

dans laquelle $R^{28}$ représente morpholinyle ou pipéridyle, $R^{31}$ représente amino, alkylamino, dialkylamino, morpholinyle ou pipéridyle, $R^{29}$, $R^{30}$ et $R^{33}$ sont identiques ou différents et chacun représente l'hydrogène ou alkyle, $R^{32}$ représente l'hydrogène, alkyle ou nitro, ou $R^{28}$ conjointement avec $R^{33}$ représente soit un élément de la formule Xa

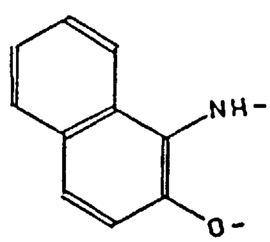

dans laquelle le groupe amino est dans la terminaison $R^{28}$, soit $R^{28}$ conjointement avec $R^{33}$ représente un élément

de la formule Xb

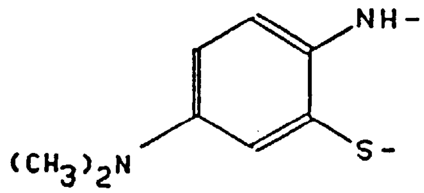

dans laquelle le groupe amino se situe dans la même terminaison $R^{28}$, 1,4-diamino-2,3,5,6-tétraméthylbenzène, 2,5-diméthyl-1,4-bis(diméthylamino)benzène, N-(4-morpholinophényl)hexahydroazépine ou le bleu de Meldola (= sels de 7-diméthylamino-1,2-benzophénoxazinium).

**2.** Procédé selon la revendication 1, dans lequel un ou plusieurs des substituants désignés $R^{29}$, $R^{30}$ et $R^{33}$ sont l'hydrogène.

**3.** Procédé selon la revendication 1 ou 2, dans lequel $R^{32}$ est l'hydrogène ou nitro.

**4.** Procédé selon la revendication 1, dans lequel le médiateur est le 1-diméthylamino-4-morpholinobenzène, 1,4-dimorpholinobenzène, 1-morpholino-4-pipéridinobenzène, 1,4-dipipéridinobenzène ou 4-nitro-3,7-bis(diméthylamino)phénothiazine (= vert de méthylène).

**5.** Procédé selon l'une quelconque des revendications précédentes, dans lequel le médiateur est présent en totalité ou en partie sous la forme cristalline dans le matériau de mise en pâte.

**6.** Utilisation de dérivés de benzène avec la formule générale X indiquée dans la revendication 1, avec les définitions des symboles indiqués ici, 1,4-diamino-2,3,5,6-tétraméthylbenzène, 2,5-diméthyl-1,4-bis(diméthylamino)benzène, N-(4-morpholinophényl)hexahydroazépine ou bleu de Meldola (= sels de 7-diméthylamino-1,2-benzophénoxazinium) en tant que médiateurs de transfert de charge.

**7.** Utilisation, selon la revendication 6, des médiateurs indiqués dans la revendication 2.

**8.** Utilisation, selon la revendication 6, des médiateurs indiqués dans la revendication 3.

**9.** Utilisation, selon la revendication 6, des médiateurs indiqués dans la revendication 4.